Europäisches Patentamt

European Patent Office    (11) Publication number:    **0 339 668**

Office européen des brevets    **A2**

(12) **EUROPEAN PATENT APPLICATION**

(21) Application number: 89107763.8    (51) Int. Cl.4: **A61K 39/29**

(22) Date of filing: 28.04.89

(30) Priority: 28.04.88 JP 106750/88

(43) Date of publication of application:
02.11.89 Bulletin 89/44

(84) Designated Contracting States:
AT BE CH DE ES FR GB GR IT LI LU NL SE

(71) Applicant: National Institute of Health
No. 10-35, Kamiosaki 2-chome
Shinagawa-ku Tokyo(JP)

Applicant: DENKA SEIKEN CO., LTD.
Taiyo Bldg. 12-1, Nihonbashi
Kabuto-cho Chuo-ku Tokyo(JP)

Applicant: CHIBA-KEN
1-1, Ichibacha-cho
Chiba-shi Chiba-ken(JP)

Applicant: Juridical Foundation The
Chemo-Sero-Therapeutic Research Institute
668, Okubo Shimizu-machi

Kumamoto-shi Kumamoto-ken(JP)

(72) Inventor: Moritsugu, Yasuo
1-14-23, Dai-machi
Hachioji-shi Tokyo(JP)
Inventor: Totsuka, Atsuko
5-16-2-107, Tamagawa-cho
Akishima-shi Tokyo(JP)
Inventor: Sato, Seiya
3-18-5, Akiba
Niitu-shi Niigata-ken(JP)
Inventor: Morita, Michio
1-10-4, Chishirodai-Higashi
Chiba-shi Chiba-ken(JP)
Inventor: Mizuno, Kyosuke
1725-1, Kamitatsuta Tatsuta-machi
Kumamoto-shi Kumamoto-ken(JP)

(74) Representative: Vossius & Partner
Siebertstrasse 4 P.O. Box 86 07 67
D-8000 München 86(DE)

(54) **Hepatitis A virus antigen.**

(57) A hepatitis A virus antigen comprising inactivated or attenuated hepatitis A virus particles is disclosed in the present application. The present antigen coprises both complete particles containing the hepatitis A virus RNA and immature empty particles free of the virus RNA. By using these two kinds of virus particles as materials for vaccines, the infection with hepatitis A can be prevented or treated. Moreover, the HAV antigen can be used as an immunogen for obtaining an anti-HAV antibody or immunoglobulin for imparting passive immunization to an objection animal.

**F I G . 2**

## Hepatitis A Virus Antigen

The present invention relates to a hepatitis A virus antigen, and more particularly to a hepatitis A virus antigen comprising an inactivated or attenuated HAV particles comprising both complete or mature particles containing hepatitis A virus RNA and immature empty particles free of the virus RNA.

Background of the invention

Hepatitis A is a disease caused by the hepatitis A virus (hereunder referred to as "HAV") and an epidemiologically and clinically important infectious disease. However, there has not yet been developed an effective means for treating hepatitis A. For this reason, preventive methods have been intensively investigated against such hepatitis A. At present globulin preparations are administered every two or three months. However, the titer of anti-HAV antibody in such a globulin preparation is reduced with the lapse of time and such a preparation must be frequently administered to achieve a desired immunization effect. Under such circumstances, development of vaccines has been strongly desired, but no vaccines have yet been put into practical use.

Experimental proliferation of HAV in cell culture was established in 1979 by Provost & Hilleman for the first time (Provost, P.J., Hilleman, M.R., Proc. Soc. Exp. Biol. Med., 1979, 160, p. 213). Thereafter, the proliferation of HAV was confirmed using several kinds of cultured cells in various parts of the world. The present inventors have established the GL 37 cell line which produces HAV in a high yield by cloning African Green monkey kidney primary cells in accordance with a colony culture technique. The present inventors have already established the KRM 003 strain, as a virus strain, which has an excellent growth property, by isolating HAV from stool of hepatitis A patients and passing it many times using the foregoing GL 37 cells as host cells. Thus, it has been possible to secure a large amount of HAV in Japan for the first time, by using the GL 37 cell line and the HAV KRM 003 strain (Summary of 33th Meeting of the Society of Japanese Virologists, p. 257 (1985)). However, it was found that approximately half of the virus particles obtained by the foregoing tissue culture technique were empty particles.

It is known that the virus belonging to the family Picorna viridae produces, during intracellular replication, complete particles containing the hepatitis A virus RNA and immature empty particles free of such virus RNA; that in particular, many enteroviruses such as poliovirus produce two kinds of particles which exhibit different antigenicity; and that only the complete particles involving the virus RNA produce neutralizing antibodies in vivo. However, the immune electron microscopy indicates that the immature empty particles and complete particles of HAV are present in the same aggregate, and therefore this suggests that they have common antigenicity. Under such circumstances, the present inventors have thought that the immature empty particles can be also used in vaccines for preventing the infection with HAV, and have examined their immunogenicity utilizing various animals. As a result, the present inventors have found out that the immature empty particles also produce neutralizing antibodies. Moreover, a marmoset immunized with such immature empty particles was protected against the challenge of virulent HAV and its serum globulin fraction exhibited a passive immunization effect. And, the present inventors have demonstrated that the immature empty particles are also useful as materials for inactivated vaccines like the complete particles (Summary of 35th Meeting of the Society of Japanese Virologists, p. 257 (1987)).

Accordingly, an object of the present invention is to provide an HAV antigen effective for preparing vaccines for preventing and treating hepatitis A.

Another object of the present invention is to provide a method for preparing hepatitis A vaccines containing both complete particles containing the HAV RNA and immature empty particles free of the HAV RNA.

A further object of the present invention is to provide an immunogen for obtaining anti-HAV immunoglobulin for inducing passive immunization.

The above objects have been accomplished by an HAV antigen comprising inactivated or attenuated HAV particles containing both complete particles containing the HAV RNA and immature empty particles free of the HAV RNA.

Fig. 1 shows fraction patterns observed when a hepatitis A virus solution is purified by sucrose density-gradient centrifugation.

Fig. 2 shows the dose-response of anti-HAV antibody producibility in SD mice immunized with a vaccine comprising formalin-inactivated complete particles and a vaccine comprising formalin-inactivated immature empty particles.

2

As an HAV antigen, HAV obtained by tissue culture is usable. HAV, which has been adapted to grow in HAV-susceptive cells, are inoculated to these cells, the infected cells are cultivated for a predetermined period of time and solubilized with a nonionic surfactant such as deoxycholic acid or NP-40 to release HAV from the cells retaining it. The solution containing HAV is extracted with an organic solvent such as chloroform or butanol after concentrating the solution by ultracentrifugation or a polyethylene glycol method. The solution is further diluted with a 2.5 M phosphate buffer (ph 7.5), mixed with a 2:1 (by volume) mixed solution of ethoxybutanol and butoxybutanol, then centrifuged to collect virus layer concentrated at the boundary between the water phase and the organic phase. The collected virus layer is resuspended in a proper buffer which contains a small amount (e.g., 0.01 to 0.001%) of a surfactant such as Tween 80 and has an approximately neutral pH (for instance, phosphate buffer or tris-HCl buffer). After the above organic solvent treatment is repeated several times, the solution is subjected to sucrose density-gradient centrifugation. After the centrifugation, the sucrose density-gradient solution is fractionated. The enzyme-linked immunosorbent assay (ELISA) technique on the HAV antigenic potency of the fractions shows bimodality. The two HAV antigen positive fractions are subjected to gel chromatography, etc. to remove sucrose and the absorption spectra within the range of from 220 to 350 nm are examined by a spectrophotometer. As a result, HAV complete particles containing the virus RNA and showing an absorption peak at 260 nm are obtained from the fraction, which is obtained by centrifugation and whose sedimentation velocity is high, while there are obtained from the fraction whose sedimentation velocity is low, HAV immature empty particles free of the virus RNA having an absorption peak at 280 nm and being found to be hollow particles having a diameter of about 27 nm when they are observed by an immune electron microscopy.

Alternatively, when gel chromatography is performed without employing sucrose density-gradient centrifugation, there are obtained HAV particles in which the complete particles containing the virus RNA and immature empty particles free of the virus RNA coexist.

As stated in the above, when various separation and purification methods for biologically active substances are combined, it is possible to obtain two kinds of highly purified HAV particles, i.e., complete particles containing the virus RNA and immature empty particles free of the virus RNA. These particles may be used as the HAV antigen of the present invention.

In addition, the complete particles containing the virus RNA and the immature empty particles free of the virus RNA are reacted with a formalin solution (37% formaldehyde solution ) diluted 1/1,000 to 1/8, 000, preferably 1/4,000 at 35 to 37° C for a certain period of time, preferably 12 to 14 days, to inactivate HAV.

When rabbits were separately and repeatedly immunized with the inactivated immature empty particles or mature particles, it was observed that the sera obtained from the rabbits reacted with both immature and mature particles and exhibited a neutralization activity to infectious HAV. Moreover, when a marmoset was immunized with inactivated immature particles, it was found that the neutralizing antibody titer was increased like the inactivated mature particles and that the marmoset was protected against the challenge with virulent HAV. Namely, it was found that the HAV complete particles and the immature empty particles free of virus RNA, which particles are used as the present HAV antigen, had the same antigenicity and that also had the same immunogenicity capable of inducing, in vivo, neutralizing antibodies resistant against virulent HAV. Thus, it is clear that both of these two kinds of virus particles can be used as vaccines for preventing the infection with hepatitis A.

In addition, the HAV antigen of the present invention may be used as an immunogen for obtaining anti-HAV immunoglobulins for imparting passive immunization to an objective animal.

The present invention will hereunder be described in more detail with reference to the following examples, and the effects practically attained by the present invention will also be discussed.


Example 1: Cultivation and Purification of HAV

African Green monkey kidney cell-derived GL 37 cells having an HAV-high producibility were infected with HAV (KRM 003 strain) which had been adapted through tissue culture so that the virus infectious dose per cell (M.O.I.) was equal to 0.1, HAV was adsorbed at 37° C for one hour, and then Eagle's-minimum essential medium (E-MEM) containing 8% fetal bovine serum (hereunder referred to as "FBS") was added thereto to perform cultivation at 37 ° C for 2 to 3 weeks. The culture medium was changed with E-MEM containing 2% FBS once a week during the cultivation, followed by, after 2 or 3 weeks, absorbing the culture medium to remove it, washing the infected cells with phosphate buffer (pH 7.2 to 7.6) and dissolving the HAV infected cells at ordinary temperature for 2 hours using tris-HCl buffer (pH 7.4) containing 0.4% deoxycholic acid, 1% NP-40 (available from NAKARAI CHEMICALS CO., LTD.) and 50 mM of EDTA. To the

3

resultant solution containing the solubilized infected GL 37 cells, there were added polyethylene glycol 6,000 (available from WAKO JUNYAKU CO., LTD.) to a final concentration of 7% and sodium chloride to a final concentration of 2.3%. The solution was stirred at 4° C for 2 to 3 hours and then allowed to stand over night. This virus solution was centrifuged at 8,000 rpm for 30 minutes and tris-HCl buffer (pH 7.4) containing 0.4% deoxycholic acid, 1% NP-40 and 50 mM of EDTA was added to the resulting sediment to redissolve the sediment. This HAV solution was centrifuged at 25,000 rpm for not less than 16 hours, followed by discarding the resulting supernatant, adding to the sediment, phosphate buffer (pH 7.2 to 7.6) in an amount ranging from 1/5 to 1/10 times the volume of the virus solution at the beginning of the purification, and then subjecting it to ultrasonication to completely dissolve the centrifugal sediment. Furthermore, the solution was centrifuged at 10,000 rpm for 15 minutes, to collect the supernatant. The supernatant contained 10 to 80 µg of HAV antigens. An equal volume of chloroform was added to this HAV solution to carry out extraction for 30 minutes, the extract was centrifuged at 2,000 rpm for 30 minutes to collect the upper aqueous phase, followed by deaerating under a reduced pressure with gently stirring, to remove chloroform present in the solution.

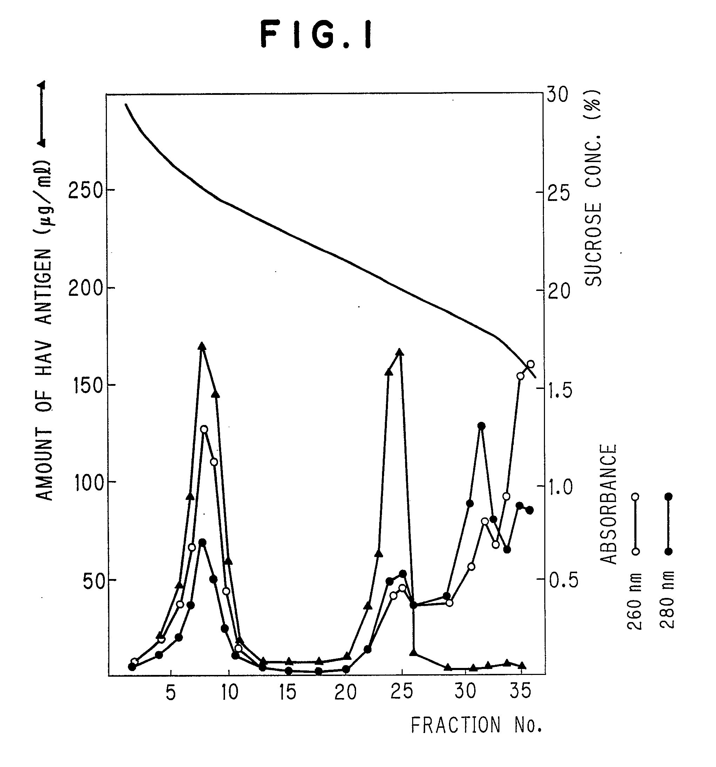

Then, RNase A (available from Sigma Co., Ltd.) was added to the solution to a final concentration of 20 µg/ml to treat the solution with the enzyme at 37° C for 1 to 2 hours, followed by further adding magnesium chloride and DNase I (available from TAKARA SHUZO Co., LTD.) at concentrations of 5 mM/ml and 20 to 40 µg/ml respectively to treat the solution at 37° C for 3 hours. Thereafter, Proteinase K (available from Merck Co., Ltd.) was added thereto at a concentration of 50 µg/ml to treat the resultant solution at 37° C for one hour. After the enzyme treatment, an equivalent amount of 2.5M phosphate buffer (pH 7.5) was added to the HAV solution, further a 2:1 mixed solution of ethoxyethanol and butoxyethanol was added thereto in an amount of 0.8 times, the volume of the virus solution, followed by gently mixing it, allowing it to stand for 10 to 30 minutes, centrifuging it at 2,000 rpm for 10 minutes, collecting the HAV layer precipitated at the boundary between the water and organic solvent phases and dissolving the HAV sediment in phosphate buffer (pH 7.2 to 7.6) containing 0.001 to 0.1% of Tween 80 (available from WAKO JUNYAKU CO., LTD.) so that the concentration of HAV antigen was 100 to 400 µ g/ml. Then, this organic solution treatment was conducted once more. The resultant HAV solution was layered on a sucrose solution having a continuous concentration gradient ranging from 10 to 40% to perform centrifugation at 25,000 rpm for 4 to 5 hours and then fractionated. The HAV antigen titer of the fractions thus obtained was determined according to a sandwich ELISA technique to select two fractions which showed two different peaks of the HAV antigen activity due to the difference in sedimentation velocity. Each fraction was chromatographed using Sephacryl S 300 (available from Pharmacia Co., Ltd.) to remove sucrose. Absorption spectra of the resultant two kinds of HAV solutions were measured at wavelength from 220 to 350 nm using a spectrophotometer. As a result, there were obtained HAV immature empty particles free of the virus RNA having an absorption spectrum at 280 nm and HAV complete particles containing the virus RNA and having an absorption spectrum at 260 nm. Fig. 1 shows the fraction patterns observed when the HAV solution was subjected to sucrose density gradient centrifugation.

Alternatively, a purified HAV solution in which the complete particles containing the virus RNA and immature empty particles free of the virus RNA coexisted was obtained by subjecting the HAV solution after the foregoing organic solvent treatment, to chromatography using Sephacryl S 400 HR (available from Pharmacia Co., Ltd.).

## Example 2: Inactivation

The HAV complete particles containing the virus RNA or HAV immature empty particles free of the virus RNA and the purified HAV solution containing both of these particles obtained by Example 1 were filtered through a filter having a pore size ranging from 50 to 200 nm to remove aggregates present therein, the resultant filtrate was mixed with an equivalent amount of formalin (37% formaldehyde) solution diluted 1/2,000 with phosphate buffer (pH 7.2 to 7.6) containing 0.002% Tween 80 to cause reaction at 37° C for 12 to 14 days. After completion of the reaction, the reaction solution was again filtered through a filter having a pore size of 200 nm to obtain a formalin-treated HAV solution.

The resultant virus solution was not capable of infecting the GL 37 cells obtained by tissue culture or non-human primates such as red-bellied tamarin, monthtached tamarin and Common marmoset susceptive to HAV. Thus, a completely inactivated HAV solution was obtained.

## Example 3: Reactivity with Anti-HAV Antibody

A vaccine comprising the formalin-inactivated immature empty particles and a vaccine comprising the formalin-inactivated complete particles obtained by Example 2 were separately inoculated into rabbits through auricular vein several times at 1 to 2 weeks intervals in an amount of not less than 100 $\mu$g of HAV antigen protein per inoculation, and about 60 to 90 days after the first inoculation, these rabbits were bled. The neutralizing antibody activity of the resultant serum to infectious HAV was determined, and its reactivity to the formalin-inactivated immature empty particles and formalin-inactivated complete particles was evaluated according to a sandwich ELISA technique. The results are listed in Table I below. As seen from the results listed in Table I, not only the serum obtained from the rabbits inoculated with vaccine comprising the formalin-inactivated immature empty particles but also that from the rabbits inoculated with the vaccine comprising the formalin-inactivatd complete particles showed a neutralizing activity to the infectious HAV. Moreover, it was confirmed that the sera obtained from these rabbits were reacted with both the formalin-inactivated immature empty particles and the formalin-inactivated complete particles. This suggests that the antigenicity of the formalin-inactivated immature empty particles is identical with that of the formalin-inactivated complete particles.

Table I

| Immunogen | Neutralizing Antibody titer | Antibody Titer by Sandwich ELISA Method | |
|---|---|---|---|
| | | Anti-Comp. Part. | Anti-empty Part. |
| Comp. Part. Empty Part. | 398 IU 32 IU | 1585 IU 200 IU | 1585 IU 200 IU |
| * All of the antibody titer values in Table I are expressed in terms of a value relative to 100 IU set for U.S. BOB Reference immunoglobulin No. 1. ** In Table I, "Comp." and "Part." mean complete and particles respectively. | | | |

Example 4: Anti-HAV Antibody Producibility

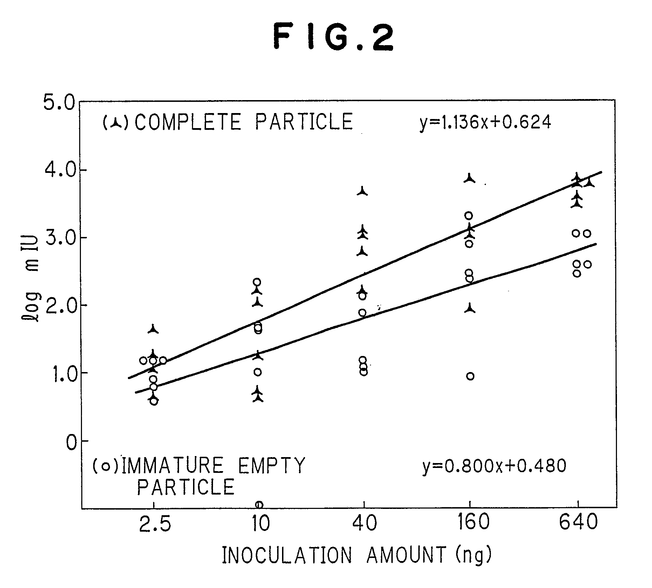

The vaccine comprising the formalin-inactivated immature empty particles and the vaccine comprising the formalin-inactivated complete particles, as prepared by Example 2, were diluted with physiological saline to prepare three kinds of samples and 1 ml of each sample was subcutaneously inoculated into 5-week-old SD rats. The rats were bled 6 week after the inoculation, followed by determining anti-HAV antibody titer in accordance with a sandwich ELISA technique and analyzing the dose-response regression formula. Both of these vaccines showed a linearlity between the inoculation amount and the antibody titer and the analysis of variance indicated the parallelism between these vaccines. In addition, the immature empty particles showed the relative potency of the order of about 1/5 times that of the complete particles. The results are shown in Fig. 2.

Example 5: Infection Inhibitory Effect in Non-Human Primate

The vaccine comprising the formalin-inactivated immature empty particles and the vaccine comprising the formalin-inactivated complete particles, which were obtained by Example 2, were respectively and subcutaneously inoculated two times into non-human primates susceptive to HAV, at 4 weeks intervals, the amount of the HAV antigen protein per inoculation being 140 ng. 14 Weeks after the second inoculation, the animals were orally challenged with a 1,000-marmoset infectious amount of virulent HAV. After the challenge, the blood was collected from the animals once a month, the anti-HAV antibody titer of the sera was determined by a competitive ELISA technique, and a serum transaminase activity (glutamic acid oxaloacetic acid transaminase, glutamic acid pyruvic acid transaminase) was also determined. In addition, the amount of the HAV antigen excreted into feces every day was also determined by a sandwich ELISA technique. As a result, the non-human primates, which were inoculated with the vaccine comprising the formalin-inactivated immature empty particles or the formalin-inactivated complete particles, produced the anti-HAV antibody 3 weeks after the first inoculation of the vaccine, but the increase in transaminase activity of the serum was not observed in all of the animals examined. Moreover, even after being orally challenged with virulent HAV, the non-human primates, which were inoculated with these vaccines, did not show the increase in the transaminase activity of the serum, and the increase in the anti-HAV antibody of the serum and the excretion of HAV into the feces were not observed at all. This clearly shows that the infection with hepatitis A was prevented. The results obtained are summarized in Table II.

Table II

| Vaccine | No. of Animal | Presence or Absence of Infection After Challenge with Virulent HAV or Its Degree | | | |
|---|---|---|---|---|---|
| | | I | II | III | IV |
| Comp. Part. | 4 | 1 | 1 | 2 | 0 |
| Empty Part. | 2 | 1 | 0 | 1 | 0 |
| (Natural Inf.) | 2 | 2 | 0 | 0 | 0 |
| (not Inocul.) | 3 | 0 | 0 | 0 | 3 |
| I: Not infected. | | | | | |
| II: After the challenge, a slight increase in anti-HAV antibody was observed, but infection was not caused. The increase in transaminase was not observed. | | | | | |
| III: After challenge, a relatively intensive increase in anti-HAV antibody was observed and a small amount of HAV was excreted in feces. The increase in transaminase was not observed. | | | | | |
| IV: Typical strong infection. | | | | | |

Example 6: Passive Immunization Effect in Non-human Primate

Non-human primates, inoculated with the vaccine comprising the formalin-inactivated immature empty particles or the vaccine comprising the formalin-inactivated complete particles described in Example 5, were bled and immunoglobulin fractions were obtained from the collected blood according to an ethanol precipitation or ammonium sulfate precipitation technique. The anti-HAV antibody titer of the resultant immunoglobulin fractions was determined by a competitive ELISA technique. An amount (corresponding to 50 IU expressed in terms of a titer relative to 100 IU set for U.S. Reference immunoglobulin G No.1) of each immunoglobulin fraction was intramuscularly inoculated into the muscle quadriceps femoris of non-human primates which did not have anti-HAV antibody. 10 Days after the inoculation, the non-human primates inoculated with the immunoglobulin fractions were orally challenged with a 1,000-marmoset infectious amount of a virulent HAV solution. Thereafter, the variation of the amount of anti-HAV antibody in blood, the activity of serum transaminase and the amount of HAV antigens excreted into feces were determined to examine whether or not the animal was infected with hepatitis A. As a result, all of the non-human primates did not show any change which indicated the infection with hepatitis A. In other words, the immunoglobulin fraction obtained from the marmosets inoculated with the vaccine comprising the formalin-inactivated immature empty particles also protected the animal against the infection caused by the challenge with the virulent HAV, like the immunoglobulin fraction obtained fro the marmosets in convalescence from HAV infection and that obtained from the marmosets inoculated with the vaccine comprising the formalin-inactivated complete particles. Therefore, the vaccine comprising the formalin-inactivatd immature empty particles is demonstrated to exhibit a remarkable passive immunization effect.

6

## Claims

1. A hepatitis A virus antigen comprising inactivated or attenuated hepatitis A virus particles, said particles being complete particles containing the hepatitis A virus RNA or immature empty particles free of the virus RNA or a mixture thereof.

2. The hepatitis A virus antigen of claim 1 wherein the hepatitis A virus particles are inactivated with formaldehyde.

3. The hepatitis A virus antigen of claim 1 wherein the hepatitis A virus particles are obtained by extracting hepatitis A virus proliferated in a tissue culture with ethoxyethanol and butoxyethanol, subjecting the extract to sucrose density-gradient centrifugation, and then selecting bands which have absorption peaks at 260 and 280 nm, respectively.

4. The hepatitis A virus antigen of claim 1 wherein the hepatitis A virus particles are obtained by extracting hepatitis A virus proliferated in a tissue culture with ethoxyethanol and butoxyethanol, and then subjecting the extract to gel chromatography.

5. An immunogen for obtaining an anti-hepatitis A virus immunoglobulin for inducing passive immunization, said immunogen comprising inactivated or attenuated hepatitis A virus particles according to any one of claims 1 to 4.

6. The hepatitis A virus antigen of any one of claims 1 to 4 or the immunogen of claim 5 for use as a vaccine for the protection against hepatitis A virus infections.

7. The hepatitis A virus antigen of any one of claims 1 to 4 or the immunogen of claim 5 for use to induce an anti-hepatitis A antibody to obtain an immunoglobulin.

8. Vaccine for the active immunization against hepatitis A virus infections containing a hepatitis A virus antigen according to any one of claims 1 to 4 or an immunogen according to claim 5.

9. Vaccine for the passive immunization against hepatitis A virus infections containing anti-hepatitis A virus immunoglobulins obtained by the induction of an immunoresponse with a hepatitis A virus antigen according to any one of claims 1 to 4 or an immunogen according to claim 5.

# FIG. 1

# F I G.2